# EUROPÄISCHE PATENTSCHRIFT

(11) **EP 1 650 305 B1**
(45) Veröffentlichungstag und Bekanntmachung des Hinweises auf die Patenterteilung: **12.08.2009**
(21) Anmeldenummer: 06001491.7
(22) Anmeldetag: 05.07.2001
(51) Int. Cl.: C12N 15/57, C12N 9/64, C12Q 1/68, C07K 16/40, A61K 38/48, G01N 33/573

(54) **Mutanten der den Faktor VII aktivierenden Protease und Nachweisverfahren mit spezifischen Antikörpern**
Mutants of the factor VII activating protease and methods for their detection
Mutants de la protéase pour l'activation du facteur de coagulation VII

(30) Priorität: 26.07.2000 DE 10036641; 10.10.2000 DE 10050040; 21.10.2000 DE 10052319; 12.04.2001 DE 10118706
(43) Veröffentlichungstag der Anmeldung: 26.04.2006
(62) Teilanmeldung aus: 01115691.6
(73) Patentinhaber: CSL Behring GmbH, 35041 Marburg (DE)
(72) Erfinder: Römisch, Jürgen, Dr., 2440 Gramatneusiedl (AT); Stöhr, Hans-Arnold, 35083 Wetter (DE); Feussner, Annette, 35043 Marburg (DE); Lang, Wiegand, 35091 Cölbe (DE); Weimer, Thomas, Dr., 35075 Gladenbach (DE); Becker, Margret, 35043 Marburg (DE); Nerlich, Claudia, 35041 Marburg (DE); Muth-Naumann, Gudrun, 35083 Wetter (DE)

(56) Entgegenhaltungen:
- EP-A- 1 033 405
- EP-A- 1 059 359
- EP-A- 1 074 615
- EP-B- 1 182 258
- DE-A- 19 903 693
- ROEMISCH J ET AL: "A PROTEASE ISOLATED FROM HUMAN PLASMA ACTIVATING FACTOR VII INDEPENDENT OF TISSUE FACTOR" BLOOD COAGULATION & FIBRINOLYSIS, RAPID COMMUNICATIONS, OXFORD,OXFORD, GB, Bd. 10, Nr. 8, Dezember 1999 (1999-12), Seiten 471-479, XP001036685 ISSN: 0957-5235
- PRINS THEO W ET AL: "Cloning and characterization of a glutathione S-transferase homologue from the plant pathogenic fungus Botrytis cinerea" MOLECULAR PLANT PATHOLOGY, Bd. 1, Nr. 3, Mai 2000 (2000-05), Seiten 169-178, XP007900023 ISSN: 1464-6722
- CHOI-MIURA N ET AL.: "Purification and characterization of a novel hyaluronan-binding protein (PHBP) from human plasma: It has three EGF, a kringle and a serine protease domain, similar to hepatocyte growth factor activator" JOURNAL OF BIOCHEMISTRY, Bd. 119, 1996, Seiten 1157-1165, XP000960750 Tokyo

### SEQUENCE LISTING

<110> ZLB Behring GmbH
<120> Mutanten der den Faktor VII aktivierenden Protease
<130> 2000/A008-A7
<150> DE 10036641.4
   <151> 2000-07-26
<160> 4
<170> PatentIn version 3.3
<210> 1
   <211> 1683
   <212> DNA
   <213> Homo sapiens
<400> 1
   atgtttgcca ggatgtctga tctccatgtt ctgctgttaa tggctctggt gggaaagaca      60
   gcctgtgggt tctccctgat gtctttattg gaaagcctgg acccagactg gacccctgac     120
   cagtatgatt acagctacga ggattataat caggaagaga acaccagtag cacacttacc     180
   catgctgaga atcctgactg gtactacact gaggaccaag ctgatccatg ccagcccaac     240
   ccctgtgaac acggtgggga ctgcctcgtc catgggagca ccttcacatg cagctgcctg     300
   gctcctttct ctgggaataa gtgtcagaaa gtgcaaaata cgtgcaagga caacccatgt     360
   ggccggggcc aatgtctcat tacccagagt cctccctact accgctgtgt ctgtaaacac     420
   ccttacacag gtcccagctg ctcccaagtg gttcctgtat gcaggccaaa cccctgccag     480
   aatggggcta cctgctcccg gcataagcgg agatccaagt tcacctgtgc ctgtcccgac     540
   cagttcaagg ggaaattctg tgaaataggt tctgatgact gctatgttgg cgatggctac     600
   tcttaccgag ggaaaatgaa taggacagtc aaccagcatg cgtgccttta ctggaactcc     660
   cacctcctct tgcaggagaa ttacaacatg tttatggagg atgctgaaac ccatgggatt     720
   ggggaacaca atttctgcag aaacccagat gcggacgaaa agccctggtg ctttattaaa     780
   gttaccaatg acaaggtgaa atgggaatac tgtgatgtct cagcctgctc agcccaggac     840
   gttgcctacc cagaggaaag ccccactgag ccatcaacca agcttccggg gtttgactcc     900
   tgtggaaaga ctgagatagc agagaggaag atcaagagaa tctatggagg ctttaagagc     960
   acggcgggca agcacccatg gcaggcgtcc ctccagtcct cgctgcctct gaccatctcc    1020
   atgccccagg gccacttctg tggtggggcg ctgatccacc cctgctgggt gctcactgct    1080
   gcccactgca ccgacataaa aaccagacat ctaaaggtgg tgctagggga ccaggacctg    1140
   aagaaagaag aatttcatga gcagagcttt agggtggaga agatattcaa gtacagccac    1200
   tacaatgaaa gagatgagat tccccacaat gatattgcat tgctcaagtt aaagccagtg    1260
   gatggtcact gtgctctaga atccaaatac gtgaagactg tgtgcttgcc tgatgggtcc    1320
   tttccctctg ggagtgagtg ccacatctct ggctggggtg ttacagaaac aggaaaaggg    1380
   tcccgccagc tcctggatgc caaagtcaag ctgattgcca acactttgtg caactcccgc    1440
   caactctatg accacatgat tgatgacagt atgatctgtg caggaaatct tcagaaacct   1500
   gggcaagaca cctgccaggg tgactctgga ggccccctga cctgtgagaa ggacggcacc   1560
   tactacgtct atgggatagt gagctggggc ctggagtgtg ggaagaggcc aggggtctac   1620
   acccaagtta ccaaattcct gaattggatc aaagccacca tcaaaagtga aagtggcttc   1680
   taa                                                                 1683
<210> 2
   <211> 1683
   <212> DNA
   <213> Homo sapiens
<400> 2
   atgtttgcca ggatgtctga tctccatgtt ctgctgttaa tggctctggt gggaaagaca     60
   gcctgtgggt tctccctgat gtctttattg gaaagcctgg acccagactg gacccctgac    120
   cagtatgatt acagctacga ggattataat caggaagaga acaccagtag cacacttacc    180
   catgctgaga atcctgactg gtactacact gaggaccaag ctgatccatg ccagcccaac    240
   ccctgtgaac acggtgggga ctgcctcgtc catgggagca ccttcacatg cagctgcctg    300
   gctcctttct ctgggaataa gtgtcagaaa gtgcaaaata cgtgcaagga caacccatgt    360
   ggccggggcc aatgtctcat tacccagagt cctccctact accgctgtgt ctgtaaacac    420
   ccttacacag gtcccagctg ctcccaagtg gttcctgtat gcaggccaaa cccctgccag    480
   aatggggcta cctgctcccg gcataagcgg agatccaagt tcacctgtgc ctgtcccgac    540
   cagttcaagg ggaaattctg tgaaataggt tctgatgact gctatgttgg cgatggctac    600
   tcttaccgag ggaaaatgaa taggacagtc aaccagcatg cgtgccttta ctggaactcc    660
   cacctcctct tgcaggagaa ttacaacatg tttatggagg atgctgaaac ccatgggatt    720
   ggggaacaca atttctgcag aaacccagat gcggacgaaa agccctggtg ctttattaaa    780
   gttaccaatg acaaggtgaa atgggaatac tgtgatgtct cagcctgctc agcccaggac    840
   gttgcctacc cagaggaaag ccccactgag ccatcaacca agcttccggg gtttgactcc    900
   tgtggaaaga ctgagatagc agagaggaag atcaagagaa tctatggagg ctttaagagc    960
   acggcgggca agcacccatg gcaggcgtcc ctccagtcct cgctgcctct gaccatctcc   1020
   atgccccagg gccacttctg tggtggggcg ctgatccacc cctgctgggt gctcactgct   1080
   gcccactgca ccgacataaa aaccagacat ctaaaggtgg tgctagggga ccaggacctg   1140
   aagaaagaag aatttcatga gcagagcttt agggtgcaga agatattcaa gtacagccac   1200
   tacaatgaaa gagatgagat tccccacaat gatattgcat tgctcaagtt aaagccagtg   1260
   gatggtcact gtgctctaga atccaaatac gtgaagactg tgtgcttgcc tgatgggtcc   1320
   tttccctctg ggagtgagtg ccacatctct ggctggggtg ttacagaaac aggaaaaggg   1380
   tcccgccagc tcctggatgc caaagtcaag ctgattgcca acactttgtg caactcccgc   1440
   caactctatg accacatgat tgatgacagt atgatctgtg caggaaatct tcagaaacct   1500
   gggcaagaca cctgccaggg tgactctgga ggccccctga cctgtgagaa ggacggcacc   1560
   tactacgtct atgggatagt gagctggggc ctggagtgtg agaagaggcc aggggtctac   1620
   acccaagtta ccaaattcct gaattggatc aaagccacca tcaaaagtga aagtggcttc   1680
   taa                                                                 1683
<210> 3
   <211> 560
   <212> PRT
   <213> Homo sapiens
<400> 3
   Met Phe Ala Arg Met Ser Asp Leu His Val Leu Leu Leu Met Ala Leu
   1               5                   10                  15
   Val Gly Lys Thr Ala Cys Gly Phe Ser Leu Met Ser Leu Leu Glu Ser
               20                  25                  30
   Leu Asp Pro Asp Trp Thr Pro Asp Gln Tyr Asp Tyr Ser Tyr Glu Asp
           35                  40                  45
   Tyr Asn Gln Glu Glu Asn Thr Ser Ser Thr Leu Thr His Ala Glu Asn
       50                  55                  60
   Pro Asp Trp Tyr Tyr Thr Glu Asp Gln Ala Asp Pro Cys Gln Pro Asn
   65                  70                  75                  80
   Pro Cys Glu His Gly Gly Asp Cys Leu Val His Gly Ser Thr Phe Thr
                   85                  90                  95
   Cys Ser Cys Leu Ala Pro Phe Ser Gly Asn Lys Cys Gln Lys Val Gln
               100                 105                 110
   Asn Thr Cys Lys Asp Asn Pro Cys Gly Arg Gly Gln Cys Leu Ile Thr
           115                 120                 125
   Gln Ser Pro Pro Tyr Tyr Arg Cys Val Cys Lys His Pro Tyr Thr Gly
       130                 135                 140
   Pro Ser Cys Ser Gln Val Val Pro Val Cys Arg Pro Asn Pro Cys Gln
   145                 150                 155                 160
   Asn Gly Ala Thr Cys Ser Arg His Lys Arg Arg Ser Lys Phe Thr Cys
                   165                 170                 175
   Ala Cys Pro Asp Gln Phe Lys Gly Lys Phe Cys Glu Ile Gly Ser Asp
               180                 185                 190
   Asp Cys Tyr Val Gly Asp Gly Tyr Ser Tyr Arg Gly Lys Met Asn Arg
           195                 200                 205
   Thr Val Asn Gln His Ala Cys Leu Tyr Trp Asn Ser His Leu Leu Leu
       210                 215                 220
   Gln Glu Asn Tyr Asn Met Phe Met Glu Asp Ala Glu Thr His Gly Ile
   225                 230                 235                 240
   Gly Glu His Asn Phe Cys Arg Asn Pro Asp Ala Asp Glu Lys Pro Trp
                   245                 250                 255
   Cys Phe Ile Lys Val Thr Asn Asp Lys Val Lys Trp Glu Tyr Cys Asp
               260                 265                 270
   Val Ser Ala Cys Ser Ala Gln Asp Val Ala Tyr Pro Glu Glu Ser Pro
           275                 280                 285
   Thr Glu Pro Ser Thr Lys Leu Pro Gly Phe Asp Ser Cys Gly Lys Thr
       290                 295                 300
   Glu Ile Ala Glu Arg Lys Ile Lys Arg Ile Tyr Gly Gly Phe Lys Ser
   305                 310                 315                 320
   Thr Ala Gly Lys His Pro Trp Gln Ala Ser Leu Gln Ser Ser Leu Pro
                   325                 330                 335
   Leu Thr Ile Ser Met Pro Gln Gly His Phe Cys Gly Gly Ala Leu Ile
               340                 345                 350
   His Pro Cys Trp Val Leu Thr Ala Ala His Cys Thr Asp Ile Lys Thr
           355                 360                 365
   Arg His Leu Lys Val Val Leu Gly Asp Gln Asp Leu Lys Lys Glu Glu
       370                 375                 380
   Phe His Glu Gln Ser Phe Arg Val Glu Lys Ile Phe Lys Tyr Ser His
   385                 390                 395                 400
   Tyr Asn Glu Arg Asp Glu Ile Pro His Asn Asp Ile Ala Leu Leu Lys
                   405                 410                 415
   Leu Lys Pro Val Asp Gly His Cys Ala Leu Glu Ser Lys Tyr Val Lys
               420                 425                 430
   Thr Val Cys Leu Pro Asp Gly Ser Phe Pro Ser Gly Ser Glu Cys His
           435                 440                 445
   Ile Ser Gly Trp Gly Val Thr Glu Thr Gly Lys Gly Ser Arg Gln Leu
       450                 455                 460
   Leu Asp Ala Lys Val Lys Leu Ile Ala Asn Thr Leu Cys Asn Ser Arg
   465                 470                 475                 480
   Gln Leu Tyr Asp His Met Ile Asp Asp Ser Met Ile Cys Ala Gly Asn
   485 490 495
   Leu Gln Lys Pro Gly Gln Asp Thr Cys Gln Gly Asp Ser Gly Gly Pro
   500 505 510
   Leu Thr Cys Glu Lys Asp Gly Thr Tyr Tyr Val Tyr Gly Ile Val Ser
   515 520 525
   Trp Gly Leu Glu Cys Gly Lys Arg Pro Gly Val Tyr Thr Gln Val Thr
   530 535 540
   Lys Phe Leu Asn Trp Ile Lys Ala Thr Ile Lys Ser Glu Ser Gly Phe
   545 550 555 560
<210> 4
   <211> 560
   <212> PRT
   <213> Homo sapiens
<400> 4
   Met Phe Ala Arg Met Ser Asp Leu His Val Leu Leu Leu Met Ala Leu
   1               5                   10                  15
   Val Gly Lys Thr Ala Cys Gly Phe Ser Leu Met Ser Leu Leu Glu Ser
               20                  25                  30
   Leu Asp Pro Asp Trp Thr Pro Asp Gln Tyr Asp Tyr Ser Tyr Glu Asp
           35                  40                  45
   Tyr Asn Gln Glu Glu Asn Thr Ser Ser Thr Leu Thr His Ala Glu Asn
       50                  55                  60
   Pro Asp Trp Tyr Tyr Thr Glu Asp Gln Ala Asp Pro Cys Gln Pro Asn
   65                  70                  75                  80
   Pro Cys Glu His Gly Gly Asp Cys Leu Val His Gly Ser Thr Phe Thr
                   85                  90                  95
   Cys Ser Cys Leu Ala Pro Phe Ser Gly Asn Lys Cys Gln Lys Val Gln
               100                 105                 110
   Asn Thr Cys Lys Asp Asn Pro Cys Gly Arg Gly Gln Cys Leu Ile Thr
           115                 120                 125
   Gln Ser Pro Pro Tyr Tyr Arg Cys Val Cys Lys His Pro Tyr Thr Gly
       130                 135                 140
   Pro Ser Cys Ser Gln Val Val Pro Val Cys Arg Pro Asn Pro Cys Gln
   145                 150                 155                 160
   Asn Gly Ala Thr Cys Ser Arg His Lys Arg Arg Ser Lys Phe Thr Cys
   165 170 175
   Ala Cys Pro Asp Gln Phe Lys Gly Lys Phe Cys Glu Ile Gly Ser Asp
   180 185 190
   Asp Cys Tyr Val Gly Asp Gly Tyr Ser Tyr Arg Gly Lys Met Asn Arg
   195 200 205
   Thr Val Asn Gln His Ala Cys Leu Tyr Trp Asn Ser His Leu Leu Leu
   210 215 220
   Gln Glu Asn Tyr Asn Met Phe Met Glu Asp Ala Glu Thr His Gly Ile
   225 230 235 240
   Gly Glu His Asn Phe Cys Arg Asn Pro Asp Ala Asp Glu Lys Pro Trp
   245 250 255
   Cys Phe Ile Lys Val Thr Asn Asp Lys Val Lys Trp Glu Tyr Cys Asp
   260 265 270
   Val Ser Ala Cys Ser Ala Gln Asp Val Ala Tyr Pro Glu Glu Ser Pro
   275 280 285
   Thr Glu Pro Ser Thr Lys Leu Pro Gly Phe Asp Ser Cys Gly Lys Thr
   290 295 300
   Glu Ile Ala Glu Arg Lys Ile Lys Arg Ile Tyr Gly Gly Phe Lys Ser
   305 310 315 320
   Thr Ala Gly Lys His Pro Trp Gln Ala Ser Leu Gln Ser Ser Leu Pro
   325 330 335
   Leu Thr Ile Ser Met Pro Gln Gly His Phe Cys Gly Gly Ala Leu Ile
   340 345 350
   His Pro Cys Trp Val Leu Thr Ala Ala His Cys Thr Asp Ile Lys Thr
   355 360 365
   Arg His Leu Lys Val Val Leu Gly Asp Gln Asp Leu Lys Lys Glu Glu
   370 375 380
   Phe His Glu Gln Ser Phe Arg Val Gln Lys Ile Phe Lys Tyr Ser His
   385 390 395 400
   Tyr Asn Glu Arg Asp Glu Ile Pro His Asn Asp Ile Ala Leu Leu Lys
   405 410 415
   Leu Lys Pro Val Asp Gly His Cys Ala Leu Glu Ser Lys Tyr Val Lys
   420 425 430
   Thr Val Cys Leu Pro Asp Gly Ser Phe Pro Ser Gly Ser Glu Cys His
           435                     440                     445
   Ile Ser Gly Trp Gly Val Thr Glu Thr Gly Lys Gly Ser Arg Gln Leu
       450                     455                     460
   Leu Asp Ala Lys Val Lys Leu Ile Ala Asn Thr Leu Cys Asn Ser Arg
   465                 470                 475                 480
   Gln Leu Tyr Asp His Met Ile Asp Asp Ser Met Ile Cys Ala Gly Asn
                   485                 490                 495
   Leu Gln Lys Pro Gly Gln Asp Thr Cys Gln Gly Asp Ser Gly Gly Pro
               500                 505                 510
   Leu Thr Cys Glu Lys Asp Gly Thr Tyr Tyr Val Tyr Gly Ile Val Ser
           515                 520                 525
   Trp Gly Leu Glu Cys Glu Lys Arg Pro Gly Val Tyr Thr Gln Val Thr
       530                 535                 540
   Lys Phe Leu Asn Trp Ile Lys Ala Thr Ile Lys Ser Glu Ser Gly Phe
   545                 550                 555                 560

## Beschreibung

Die Erfindung betrifft Verfahren zum Nachweis der dem Blut gerinnungsfaktor VII aktivierenden Protease und ihren Mutanten auf Protein- Ebene oder in einer Gewebeprobe sowie spezifische Antikörper für diese Nachweisverfahren.

Aus der deutschen Patentanmeldung 199 03 693.4 ist bereits eine aus dem Blutplasma isolierte Protease bekannt, die den Gerinnungsfaktor VII aktivieren kann. Aufgrund dieses ersten Befundes wurde sie als Faktor VII aktivierende Protease (FSAP) bezeichnet. Detaillierte Untersuchungen zeigten, dass FSAP auch ein potenter Aktivator von Einketten-Plasminogenaktivatoren ist wie Prourokinase oder Einketten-Gewebe-Plasminogenaktivator (sct-PA). Aufgrund dieser Eigenschaften wurden Anwendungsmöglichkeiten von FSAP beschrieben, beispielsweise ihre Anwendung als gerinnungsförderndes Mittel basierend auf der durch FVII-Aktivierung unterstützten Beschleunigung der Coagulation. Allein oder in Kombination mit Plasminogenaktivatoren kann FSAP auch zur Fibrinolyse Anwendung finden, beispielsweise bei thrombotischen Komplikationen.

Wie in den deutschen Patentanmeldungen 199 03 693.4 und 199 26 531.3 beschrieben, wurden Tests zur Detektion der Protease entwickelt, die sowohl die Quantifizierung des FSAP Antigengehaltes sowie deren Aktivität zum Beispiel im Plasma ermöglichen. Die Antigenbestimmung wird dabei vorzugsweise mittels eines ELISA-Tests durchgeführt. Die FSAP-Aktivität kann - wie in der deutschen Patentanmeldung 199 26 531.3 beschrieben - durch Quantifizierung der Aktivierung von Prourokinase zur Urokinase und deren Umsetzung mit einem chromogenen Substrat mit anschließender Differenzmessung der Extinktion bestimmt werden. Ein überraschender Befund bei Durchführung dieses Aktivitätstestes war, dass das aus zum Beispiel Plasma isolierte FSAP Proenzym bei den gewählten Inkubationsbedingungen aktiviert wurde und so die Aktivierung der Prourokinase ermöglichte. Neuere Untersuchungen haben gezeigt, dass FSAP durch Eigenaktivierung in die aktivierte Form überführt wird und so beispielsweise Prourokinase oder den FVII aktivieren kann. Dies wird durch die oben genannten Inkubationsbedingungen, nämlich neutraler bis alkalischer pH-Wert, Kalziumionen und Heparin noch unterstützt. Zudem weisen jüngste Ergebnisse darauf hin, dass Prourokinase/Urokinase (oder Ein- und Zweiketten-tPA) selbst eine Aktivierung von Einketten-FSAP hervorrufen oder unterstützen.

Unter Anwendung der beiden vorstehend genannten Testsysteme, nämlich dem ELISA- und dem Prourokinase-Aktivierungstest, wurden mehr als 180 Plasmen gesunder Blutspender untersucht. Dabei zeigte sich, dass 5 bis 10% aller Proben gegenüber einem Plasmapool (aus mehr als 100 gesunden Spendern) oder dem Durchschnitt des gesamten Testkollektives eine deutlich erniedrigte Potenz der durch FSAP bewirkten Prourokinase-Aktivierung aufweisen.

Dagegen wurden in der Mehrzahl dieser Spender (mit erniedrigter Aktivität) durchschnittliche FSAP-Antigenwerte gemessen. Es wird daher vermutet, dass in den untersuchten Blutproben eine oder mehrere Modifikationen des FSAP enthalten sein könnten, die eingeschränkte oder fehlende Aktivitäten zur Folge hätten. Dies könnte in Polymorphismen in der Bevölkerung, also einer oder mehrerer Mutationen in den FSAP-Strukturen, die sich in einer Änderung der FSAP-Aminosäuresequenz zeigen, begründet sein, wie schon in der deutschen Patentanmeldung 199 26 531.3 vermutet wurde. Die in der Regel um 50 bis 70% gegenüber dem Durchschnittswert aller untersuchten Spender erniedrigten Aktivitäten weisen auf eine heterozygote Mutation hin. Dies könnte sich phenotypisch durch wahrscheinlich paritätische Anwesenheit beider FSAP's, nämlich der Wildtyp-FSAP und der mutanten Variante, im Plasma ausprägen. Angenommen, die mutierte Variante hätte die Eigenschaft (nahezu) völlig eingebüßt, Prourokinase zu aktivieren, so würde im Mittel eine ungefähr halbierte Aktivität messbar werden. Darüber hinaus wurden jedoch auch schon pseudohomozygote Ausprägungen heterozygoter Mutationen anderer Proteine beschrieben, bei denen lediglich das mutierte Protein detektierbar war, welches aber als solches nur einen Teil der entsprechend detektierten biologischen Eigenschaft eingebüßt hatte.

Um auszuschließen, dass der Mangel oder die Verminderung unbekannter potentieller Kofaktoren für die festgestellte Einbuße der FSAP-Aktivität verantwortlich ist, wurden FSAP-Proben von drei Spendern gereinigt, die bei wiederholten Spenden eine signifikant erniedrigte Aktivität gezeigt hatten. Die hochgereinigten Proteine zeigten gegenüber der aus dem Plasmapool gereinigten FSAP ebenfalls eine deutlich verminderte Aktivität. Dies reduziert die Wahrscheinlichkeit eines Kofaktoreinflusses und erhöht die einer Proteinmodifikation im oben genannten Sinne. Überraschend war der Befund, dass die Potenz zur Aktivierung von Faktor VII nicht eingeschränkt zu sein scheint. Aus diesem Grunde sind solche Mutanten besonders für die oben genannte Anwendung als gerinnungsförderndes Mittel - wie in der deutschen Patentanmeldung 199 03 693.4 beschreiben - geeignet, da deren fibrinolytisches Potential offenbar limitiert ist. Diese Mutanten können basierend auf den im folgenden beschriebenen Erkenntnisse der Nukleotidsequenz-Änderungen rekombinant oder transgen hergestellt werden. Sie können aber auch ebenso wie das entsprechende FSAP-Protein (Ein- oder Zweiketten-FSAP) aus natürlichen Quellen wie Blutplasma direkt isoliert werden. In den deutschen Patentanmeldungen 199 03 693.4, 199 37 219.5 und 199 37 218.7 wurden bereits Verfahren beschrieben, die die Herstellung von FSAP erlauben, bevorzugt mit Hilfe der Immunabsorption, wie es in der deutschen Patentanmeldung 100 36 641.4 im einzelnen erläutert ist. Die bisher verwendeten monoklonalen Antikörper unterscheiden jedoch, so weit bekannt, nicht zwischen dem Wildtyp und den Mutanten des FSAP. Entsprechend können nur monoklonale Antikörper, die spezifisch mit den Mutanten reagieren, zur Herstellung der Mutanten verwendet werden. Dabei können die Antikörper durch Immunisierung mit der Mutante gewonnen werden. Außerdem können Peptide mit Proteinregionen, die den Aminosäuren 389 bis 397 (...SFRVQKIFK...) und/oder 534 bis 539 (...EKRPGV...) der SEQ. ID No. 4 des Sequenzprotokolls entsprechen, nach bekannten Methoden zur Immunisierung und Generierung entsprechender Antikörper verwendet werden. Außerdem finden diese Antikörper auch Anwendung zur spezifischen Detektion dieser Mutanten, zum Beispiel als Reagenzien in Nachweisverfahren wie ELISA, Western Blots, in der Immunhistologie oder beim Fluorescence Assisted Cell Sorting (=FACS).

Dagegen können Antikörper, die spezifisch für den FSAP-Wildtyp bzw. gegen die entsprechenden Aminosäuresequenz des Wildtyps gerichtet sind, zum Beispiel gegen die Aminosäuresequenzen 389 bis 397 (...SFRVEKIFK...) und/oder gegen die Aminosäuresequenz 534 bis 539 (...GKRPGV...) gerichtet sind vor allem in humanisierter Form als Pharmazeutikum zur prophylaktischen oder therapeutischen Inhibition der FSAP-Aktivität verwendet werden, um beispielsweise Blutungen zugrundeliegenden Hyperfibrinolysen entgegenzuwirken. Außerdem können diese Antikörper auch zur Reinigung, Detektion und Differenzierung der Wildtyp-FSAP in der oben beschriebenen Weise verwendet werden.

Die genomische Sequenz des FSAP wurde in der Genbank unter der Accession No. AC 006097 durch Abgleich mit der bekannten cDNA-Sequenz (Choi-Miura, Accession No. S 83182) identifiziert und dabei Intron- und Exon-Sequenzen abgeleitet. Insgesamt wurden 12 Primerpaare entworfen, um die kodierenden Sequenzen in spezifischen PCR-Reaktionen zusammen mit einem kleinen Teil der jeweils flankierenden Intron-Sequenzen amplizieren zu können.

Zunächst wurde die genomische DNA aus Blut von 2 Probanden mit erniedrigter und von 4 Probanden mit normaler Prourokinase-Aktivität isoliert mit allen Primerpaaren amplifiziert und anschließend unter Verwendung der PCR-Primer die DNA-Sequenz bestimmt. Das Ergebnis ist in Tabelle 1 dargestellt. Insgesamt 4 Nukleotidpositionen in der kodierenden Region waren polymorph, d.h. an diesen Stellen werden zwei Basen gleichzeitig nachgewiesen. Es ist daher davon auszugehen, dass in diesen Fällen Heterozygosität vorliegt, mit einem Wildtyp- und einem mutanten Allel. Zwei davon (an Position 183 und 957) sind Drittbasenaustausche, die nicht zu einem Aminosäureaustausch führen. Die beiden anderen, die nur in der DNA der Probanden mit erniedrigter Proruokinase-Aktivität gefunden wurden, führen zu Aminosäureaustauschen wie in Tabelle 1 dargestellt.

**Tabelle 1**

| **DNA-Sequenz an Nukleotidpositionen*** | | | | | |
|---|---|---|---|---|---|
| Proband Nr. | ProUK-Aktivität | 183 | 957 | 1177 | 1601 |
| S83182 | | T | G | G | G |
| 9689 | normal | T/C | G | G | G |
| 9690 | normal | T/C | G | G | G |
| 9704 | normal | T | G/A | G | G |
| 9706 | normal | T | G/A | G | G |
| 9714 | erniedrigt | T | G | G/C | G/A |
| 9715 | erniedrigt | T | G | G/C | G/A |

| **Aminosäure an Position*** | | | | | |
|---|---|---|---|---|---|
| Proband Nr. | ProUK-Aktivität | NT*: 183 AS*: 61 | NT: 957 AS: 319 | NT: 1177 AS: 393 | NT. 1601 AS: 534 |
| S83182 | | His | Lys | Glu | Gly |
| 9689 | normal | His | Lys | Glu | Gly |
| 9690 | normal | His | Lys | Glu | Gly |
| 9704 | normal | His | Lys | Glu | Gly |
| 9706 | normal | His | Lys | Glu | Gly |
| 9714 | erniedrigt | His | Lys | Glu/Gln | Gly/Glu |
| 9715 | erniedrigt | His | Lys | Glu/Gln | Gly/Glu |

| | | | | | |
|---|---|---|---|---|---|
| * wobei 1 das A des Initationskodons ist * NT - Nukleotidposition; AS - Aminosäureposition | | | | | |

Um die Korrelation der beiden Mutationen mit erniedrigter Prourokinase-Aktivität zu untersuchen, wurden die DNAs weiterer Personen an diesen Stellen sequenziert. Das Ergebnis ist in Tabelle 2 zusammengefasst. Alle 6 Probanden mit erniedrigter Prourokinase-Aktivität waren heterozygot an der Nukleotidposition 1601 (Gly - Glu Austausch), vier hatten zusätzlich die Heterozygosität an der Position 1177 (Glu - Gln Austausch). Keiner der insgesamt 11 Probanden mit normaler oder am unteren Normalbereich befindlicher Prourokinase-Aktivität wies die oben genannte Heterozygositäten auf. Dieses Ergebnis lässt darauf schließen, dass zumindest der Austausch an Aminosäureposition 534 ursächlich mit der erniedrigten Prourokinase-Aktivität zusammenhängt. Ob ein Aminosäureaustausch allein in der Position 393 eine Erniedrigung der Prourokinase-Aktivität zur Folge haben könnte, ist derzeit noch ungewiss.

**Tabelle 2**

| **DNA-Sequenz an Nukleotidposition** | | | |
|---|---|---|---|
| Proband Nr. | ProUK-Aktivität | 1177 | 1601 |
| 9714 | Niedrig | C/G | A/G |
| 9715 | Niedrig | C/G | A/G |
| 9802 | Niedrig | C/G | A/G |
| 10032 | Niedrig | G | A/G |
| 10039 | Niedrig | C/G | A/G |
| 10047 | Niedrig | G | A/G |
| 9698 | Unterer Normalbereich | G | G |
| 9702 | Unterer Normalbereich | G | G |
| 9711 | Unterer Normalbereich | G | G |
| 9712 | Unterer Normalbereich | G | G |
| 10038 | Unterer Normalbereich | G | G |
| 9689 | Normal | G | G |
| 9690 | Normal | G | G |
| 9704 | Normal | G | G |
| 9706 | Normal | G | G |
| 9803 | Normal | G | G |
| 10043 | Normal | G | G |

Die Nukleotidsequenz SEQ. ID No. 1 des beiliegenden Sequenzprotokolls gibt die Sequenz des Wildtyps wieder. Die DNA-Sequenz der Mutante mit beiden Austauschen an den Nukleotidpositionen 1177 und 1601 ist durch die SEQ. ID No. 2 des Sequenzprotokolls beschrieben. Die entsprechende Aminosäuresequenz des Wildtyps kann der SEQ. ID No. 3 des Sequenzprotokolls entnommen werden. Die SEQ. ID No. 4 zeigt die Aminosäuresequenz der Mutante mit den beiden Aminosäureaustauschen (Glu - Gln 393 und Gly - Glu 534).

Mit dem Auffinden der im Sequenzprotokoll genannten DNA- und Aminosäuresequenzen sind die Voraussetzungen zur Entwicklung diagnostischer Verfahren zum Erkennen von Patienten mit genetisch bedingter hetero- oder homozygoter Expression des FSAP geschaffen worden. Man kann die Mutationen entweder in der genomischen DNA oder in der daraus abgeleiteten mRNA nachweisen. Der Nachweis gelingt aber auch auf der Proteinebene mit monoklonalen oder polyklonalen Antikörpern, die gegen die Mutante mit der abgewandelten Aminosäuresequenz oder gegen den Wildtyp gerichtet sind.

Daraus ergibt sich die Notwendigkeit, hierfür geeignete Antikörper herzustellen und damit zuverlässige diagnostische Verfahren und Testsysteme zu entwickeln.

Ein Gegenstand der Erfindung sind deshalb monoklonale Antikörper gegen die den Blutgerinnungsfaktor VII aktivierende Protease und ihre Mutanten sowie ihre Verwendung zum Nachweis und zur Bestimmung der Aktivität von FSAP und ihren Mutanten.

An sich sind Testsysteme, die eine Quantifizierung sowohl des FSAP Antigengehalts als auch die Bestimmung seiner Aktivität ermöglichen, wie Western Blots oder ein Enzym-Immunoassay (ELISA) bereits aus den deutschen Patentanmeldungen 199 03 693.4 und 199 26 531.3 bekannt.

Diese Testsysteme basieren darauf, dass FSAP durch spezifische monoklonale Antikörper gebunden und/oder detektiert wird. Obwohl bei der Immunisierung beispielsweise von Mäusen häufig viele positive Klone bezüglich der Expression eines spezifischen monoklonalen Antikörpers identifiziert werden, sind doch nicht selten nur wenige davon für die vorstehend genannten Fragestellungen geeignet. Es stelle sich deshalb die Aufgabe, spezifische monoklonale Antikörper gegen die den Blutgerinnungsfaktor VII aktivierende Protease aufzufinden, die sowohl für ihre Reindarstellung, als auch für ihren qualitativen und quantitativen Nachweis als auch für ihre Aktivitätsbestimmung effektiv einsetzbar sind.

Es wurde nun gefunden, dass diesen Anforderungen in hohem Maße monoklonale Antikörper gegen die den Blutgerinnungsfaktor VII aktivierende Protease gerecht werden, die von der Hybridomazelllinie DSM ACC2453 gebildet werden. Die Hybridomazelllinie ist hinterlegt bei der Dentschen Sammlung von Mikroorganismen (DSM). Bis vor kurzem wurde FSAP überwiegend in ihrer aktivierten Form gereinigt. Konventionelle Präparationsmethoden, wie säulenchromatografische Techniken, begünstigen die rasche Aktivierung und anschließende Inaktivierung der Protease. Erfindungsgemäß ist nun die Reindarstellung der den Blutgerinnungsfaktor VII aktivierenden Protease und vor allem ihres Proenzyms mit hoher Ausbeute dadurch möglich, dass man einen der vorstehend genannten Antikörper an einen Träger koppelt, diesen mit einer die Protease oder ihr Proenzym enthaltenden Flüssigkeit equilibriert, anschließend auswäscht und dann die Protease oder ihr Proenzym durch Elution gewinnt.

Darüber hinaus eignen sich die genannten Antikörper auch zum Nachweis der den Blutgerinnungsfaktor VII aktivierenden Protease oder ihres Proenzyms mit einem Immunoassay, bei dem man
a) eine Probe, die die Protease oder ihr Proenzym enthalten soll, mit einem auf einem festen Träger fixierten ersten erfindungsgemäßen Antikörper inkubiert und auswäscht, dann einen zweiten markierten erfindungsgemäßen Antikörper zugibt und abermals auswäscht und das vom zweiten Antikörper oder anderen monoklonalen oder polyklonalen Antikörpern hervorgerufene Signal misst; oder
b) auf einem Träger die in der zu untersuchenden Probe enthaltene Protease oder ihr Proenzym fixiert, beispielsweise durch polyklonalen Antikörper gegen die Protease oder ihr Proenzym, und sie mit einem markierten erfindungsgemäßen Antikörper allein oder in Mischung mit einem unmarkierten der erfindungsgemäßen Antikörper und anschließendem Nachweis des monoklonalen Antikörpers detektiert oder
c) einen auf einem Träger fixierten erfindungsgemäßen Antikörper mit einer auf die Protease oder ihr Proenzym zu untersuchenden Probe in Gegenwart einer markierten Protease oder ihres Proenzyms versetzt und das durch die Markierung hervorgerufene Signal misst.

Eine andere Möglichkeit zum Nachweis der den Blutgerinnungsfaktor VII aktivierenden Protease oder ihres Proenzyms besteht darin, dass man den Nachweis mit der Western Blot-Methodik durch eine immunologische Reaktion mit einem markierten erfindungsgemäßen Antikörper allein oder in Mischung mit einem unmarkierten erfindungsgemäßen Antikörper und anschließendem Nachweis des monoklonalen Antikörpers, zum Beispiel durch markiertes Protein A oder G oder einen markierten, gegen den monoklonalen Antikörper gerichteten monoklonalen oder polyklonalen Antikörper durchführt.

Schließlich ist es auch möglich, die Aktivität der den Blutgerinnungsfaktor VII aktivierenden Protease oder ihres Proenzyms in Proteinlösungen dadurch zu bestimmen, dass man die die Protease und/oder ihr Proenzym enthaltende Proteinlösung an einem festen Träger inkubiert, an die zuvor ein gegen die Protease gerichteter monoklonaler erfindungsgemäßer Antikörper gekoppelt wurde und nach Auswaschen des festen Trägers die daran fixierte Protease und ihr Proenzym mit Reagentien inkubiert, die deren Aktivitätsbestimmung erlauben. Die Aktivität der Protease oder ihres Proenzyms kann dann durch eine photometrische Bestimmung der bei der Einwirkung auf chromogene Substrate auftretenden Extinktion gemessen werden.

Andere Möglichkeiten zur Bestimmung der Aktivität der Protease oder ihres Proenzyms bestehen darin, dass man
- ihre die Blutgerinnungsfaktoren VIII/VIIIa oder VNa inaktivierende Wirkung oder
- ihre die Blutgerinnungszeiten verkürzende Wirkung in globalen Gerinnungstests oder
- ihre Plasminogenaktivatoren aktivierende Wirkung oder
- ihre den Blutgerinnungsfaktor VII aktivierende Wirkung misst.

Für die Anwendung in den vorstehend genannten Verfahren eignen sich sowohl die vollständigen monoklonalen Antikörper als auch deren Fragmente wie F(ab')₂ oder Fab. Die Antikörper oder ihre Fragmente werden nach Markierung mit einer radioaktiven oder fluoreszierenden oder enzymatisch aktiven Substanz als detektierende Hilfsmittel in einem Immunoassay oder in einem Western Blot-Nachweisverfahren eingesetzt. Die unmarkierten monoklonalen Antikörper oder Fragmente können ebenfalls eingesetzt werden, jedoch wird dann die Detektion oder Immobilisierung zum Beispiel durch einen gegen den Maus-Antikörper gerichteten markierten Antikörper oder sein markiertes Fragment durchgeführt (Sandwich-Verfahren). Die erfindungsgemäßen monoklonalen Antikörper oder ihre Fragmente können allein oder als Mischung eingesetzt werden. Dies ist besonders empfehlenswert bei Western Blots, da SDS-Gelelektrophoresen häufig unter reduzierenden Bedingungen der Proben durchgeführt werden. Da die beiden Polypeptidketten der FSAP lediglich über eine Disulfidbrücke miteinander verbunden sind, zerfällt das Molekül bei der Reduktion in zwei Ketten, nämlich in die schwere und die leichte Kette, wobei die erstere vom monoklonalen Antikörper der DSM ACC2454 und die letztere vom monoklonalen Antikörper der DSM ACC2453 erkannt wird. Zur Detektion beider Ketten sind also die beiden erfindungsgemäßen monoklonalen Antikörper oder ihre Fragmente erforderlich.

Die erfindungsgemäßen monoklonalen Antikörper wurden wie folgt hergestellt und charakterisiert:

### Immunisierung

Drei weibliche Balb/c-Mäuse (ca. 6 Wochen alt) wurden mit FVII-Aktivator immunisiert. Die erste Injektion bestand aus 0,2 ml des Antigens (10 µg) gemischt mit 0,2 ml kompl. Freund's Adjuvans. In den drei folgenden Boost-Injektionen (Abstand jeweils 2 Wochen) wurde das Antigen (20 µg in 0,2 ml) ohne Adjuvans verabreicht (alle Injektionen i.p.). Die Verdünnung des Immunogens erfolgte in PBS. Nach der letzten Injektion wurden die Serum-Titer mittels indirektem ELISA bestimmt, indem eine Mikrotiterplatte mit FVII-Aktivator beschichtet wurde. Für die Fusion wurde die Maus mit dem höchsten Serum-Titer ausgewählt.

### Fusion

Etwa drei Wochen nach der letzten Applikation wurde das Antigen an drei aufeinanderfolgenden Tagen verabreicht (10 µg in 0,1 ml i.v.). Am nächsten Tag (Tag 4) wurde die Maus nach einer Blutentnahme getötet, die Milz entnommen und die Milzzellen isoliert. Die Milzzellen wurden anschließend mit der murinen Myelom-Zelllinie SP2/0-Ag 14 fusioniert. Als Fusionsreagenz wurde Polyethylenglykol 4000 (Merck) verwendet. Die Fusion wurde mit einer Modifikation der Original Köhler/Milstein-Methode durchgeführt. Die Zellen wurden auf 24 Well-Kulturplatten verteilt. Als Medium wurde Dulbecco mod. Eagle's Medium mit 10% fötalem Kälberserum und HAT zur Selektion eingesetzt. Nach etwa zwei Wochen wurden die gewachsenen Zellklone in die Vertiefungen einer 48 Weil-Platte transferiert und kodiert.

### Hybridoma-Screening

Von 1728 gewachsenen Klonen wurde der Kulturüberstand genommen und mittels ELISA auf Anwesenheit von Maus IgG getestet. Mit Hilfe von immobilisiertem FVII-Aktivator wurden Maus 1gG positive Überstände auf Spezifität geprüft (ELISA). Von den getesteten Zelllinien wurden 108 Zelllinien als spezifisch für FVII-Aktivator identifiziert und kryokonserviert.

Die Hybridoma-Zelllinie mit der Bezeichnung DSM ACC2453 wurde für weitere Untersuchungen ausgewählt. Die Spezifität der von diesen Zelllinien gebildeten Antikörper wurde mit dem BIACORE bestätigt und die Bindungskinetik untersucht. Der monoklonale Antikörper ist vom Typ des IgG1.

Mit Hilfe der beschriebenen Antikörper gegen den FSAP-Wildtyp und gegen ihre Mutanten können diagnostische Verfahren zum Nachweis der Mutanten so durchgeführt werden, dass man
a) eine Probe, die die Mutante enthalten könnte, mit einem auf einem festen Träger fixierten ersten Antikörper gemäß Anspruch 2 inkubiert und danach wäscht, dann einen zweiten, markierten Antikörper gemäß Anspruch 2 oder gegen den Wildtyp gerichteten markierten Antikörper zugibt und abermals auswäscht und das vom zweiten Antikörper hervorgerufenen Signal misst oder
b) eine Probe, die die Mutante enthalten könnte, mit einem auf einem festen Träger fixierten ersten Antikörper gegen den Wildtyp inkubiert und danach wäscht, dann einen zweiten, markierten Antikörper gemäß Anspruch 2 zugibt und abermals auswäscht und das vom zweiten Antikörper hervorgerufenen Signal misst oder
c) auf einen Träger die auf das Vorliegen der Mutante zu untersuchende Probe fixiert und sie mit einem markierten Antikörper gemäß Anspruch 2 allein oder in Mischung mit einem unmarkierten Antikörper und anschließendem Nachweis des markierten Antikörpers detektiert oder
d) einen auf einem Träger fixierten Antikörper gemäß Anspruch 2 mit einer auf das Vorliegen der Mutante zu untersuchenden Probe in Gegenwart einer markierten Mutante versetzt und das durch die Markierung hervorgerufene Signal misst.

Bevorzugt ist ein diagnostisches Verfahren, bei dem man die Aktivität der FSAP misst, indem man die die Protease enthaltende Probe an einem festen Träger inkubiert, an den zuvor ein gegen die Protease gerichteter Antikörper nach Anspruch 2 gekoppelt wurde und nach Auswaschen des festen Trägers die fixierte Protease mit Reagenzien inkubiert, die deren Aktivitätsbestimmung erlauben.

Dabei kann die Aktivität der Protease durch eine photometrische Bestimmung der bei der Einwirkung auf chromogene Substrate auftretenden Extinktion gemessen werden.

Es ist auch möglich, die Aktivität der Protease durch Messung
- ihrer die Blutgerinnungsfaktoren VIII/VIIIa und V/Va inaktivierende Wirkung oder
- ihre die Blutgerinnungszeiten verkürzende Wirkung in globalen Gerinnungstests oder
- ihrer Plasminogenaktivatoren aktivierende Wirkung oder
- ihre den Blutgerinnungsfaktor VII aktivierende Wirkung zu bestimmen.

Schließlich stehen auch Verfahren zur Verfügung, bei denen die die Plasminogenaktivatoren aktivierende Wirkung gemessen wird, durch die Aktivierung der
- Einketten-Urokinase (scuPA, single chain urokinase plasminogen activator) oder des
- Einketten-tPA (sctPA, single chain tissue plasminogen acitivator).

Zum Nachweis der für die Erniedrigung der Prourokinase-Aktivität verantwortlichen Mutationen auf DNA- und RNA-Ebene können Verfahren eingesetzt werden, wie sie auch zum Nachweis von single nucleotid polymorphisms angewendet werden, zum Beispiel
- die cDNA-Amplifikation der RNA oder die Amplifikation der genomischen DNA und ihre anschließende Sequenzierung;
- der Mutationsnachweis auf Ebene der cDNA oder genomischen DNA oder deren Amplifikation durch
- die Hybridisierung mit sequenzspezifischen Sonden, die auch Markierungen zum Nachweis tragen können wie Enzyme, alkalische Phosphatase, HRP und deren Substrate, Fluoreszenzfarbstoffe, auch Reporter-Quencher-Paare (wie zum Beispiel Scorpions, Molecular Beacons, TaqMan-Sonden), radioaktive Atome, Chromophore, Chemo- und Elektrochemolumineszenzmarkierungen) oder
- durch Verfahren wie die selektive 2'-Amin-Acylierung, die elektrochemische Oxidation von Nukleinsäuren, durch "minor groove binder" Oligonukleotid-Konjugate oder durch die HPLC.

Auf der Grundlage der Untersuchungsergebnisse, die durch die vorstehend genannten Antigen- und Aktivitätstests erhalten wurden, konnten drei Gruppen von gesunden Spendern hinsichtlich potentieller Mutationen auf genomischer Ebene untersucht werden. Dazu wurde den Spendern Blut entnommen, und die Blutzellen durch Zentrifugation vom Plasma getrennt. Die Plasmen wurden dann zur Quantifizierung der FSAP-Antigen- und Aktivitätsspiegel verwendet und entsprechend den letzteren in drei Gruppen unterteilt, nämlich in "hoch/durchschnittlich", "durchschnittlich/erniedrigt" und "signifikant erniedrigt". Die gewonnenen Blutzellen wurden dann zur DNA/RNA-Extraktion verwendet und daraus die auf Seite 5 und in Tabelle 1 dargestellten Befunde ermittelt.

Basierend auf den vorliegenden Ergebnissen ist nunmehr die rasche Detektion einer oder beider beschriebenen Mutationen, gleichgültig ob hetero- oder homozygoten Genotyps, auf der Ebene der entsprechenden FSAP-Nukleotidsequenz möglich, Während die vorstehend genannten Antigen- und Aktivitätstests in gesundem Zustand eines Spenders durchaus den Genotyp widerspiegeln, kann dies bei Einflüssen auf die FSAP-Plasmaspiegel schwierig oder unmöglich werden. So können Parameter wie hormonelle Schwankungen, Lebensstil usw. besonders aber Krankheitszustände Antigen- und/oder Aktivitätsspiegel mehr oder minder stark beeinflussen. Wie in der deutschen Patentanmeldung 199 26 531.3 beschrieben, kann bei einem Herzinfarkt die messbare FSAP-Aktivität bei kaum erhöhtem Antigengehalt deutlich gegenüber dem Normalwert ansteigen, wodurch Spender, die im gesunden Zustand eine erniedrigte FSAP-Aktivität aufweisen, nun als "durchschnittlich" erscheinen.

Beispielsweise sind Untersuchungen, ob Patienten mit FSAP-Mutation ein erhöhtes Risiko haben, thrombotische Komplikationen wie Herzinfarkte zu erleiden, aufgrund der vorstehend genannten Beschränkungen nur schwer möglich. Dagegen können beispielsweise Leberinsuffizienzen zu erniedrigten Plasmaspiegeln führen, was ebenfalls zu Missinterpretationen der "wahren" genetischen Prädispositon führen kann. Ein Test auf FSAP-Mutation auf DNA/RNA-Ebene ist dagegen von temporären Ereignissen unabhängig. Die Kombination aller genannten Assays ermöglicht ein komplettes Bild des Spenders/Patienten, nämlich die Beurteilung einer potentiellen Mutation und des akuten Zustandes hinsichtlich einer Beeinflussung des Antigen-Aktivitätsverhältnisses. Daraus können prophylaktische und therapeutische Maßnahmen resultieren.

Wie vorstehend beschrieben, wurden bisher ausschließlich heterozygote Blutspender gefunden, deren Blutplasma zu etwa 50% die normale FSAP und zu etwa 50% die FSAP-Mutante enthält. Hieraus ergibt sich ein um etwa 50% erniedrigter Aktivitätsspiegel von Plasmen, in denen beide Arten von FSAP-Molekülen vorkommen. Plasmapools, die aus dem Blut von 100 und mehr Spendern gewonnen worden sind, enthalten demnach auch je nach Population 5 bis 10% der FSAP-Mutanten. Hieraus ergibt sich bei Bluttransfusionen eine entsprechende Wahrscheinlichkeit, Blutplasma eines Spenders zu bekommen, welches die FSAP-Mutante enthält. Wird ein diese Mutante enthaltendes Blut einem Empfänger verabreicht, der diese Mutante nicht bildet, dann können diese von ihm als fremd erkannt und entsprechende Antikörper ausgebildet werden. Bei einer späteren Folgeapplikation mit der FSAP-Mutante kann es zu immunologischen Reaktionen des Empfängers mit den dem Fachmann vertrauten Nebenwirkungen kommen.

Umgekehrt wird bei einem homozygoten Blutempfänger, der lediglich die FSAP-Mutante, nicht aber die normale FSAP bildet, diese als "fremd" erkannt und entsprechende Antikörper dagegen gebildet.

FSAP beeinflusst die Hämostase und die damit verbundenen zellulären Prozesse. Durch Involvierung in die Blutgerinnung und/oder die Fibrinolyse beeinflusst es auch die Wundheilungsreaktion. Außerdem kann die FSAP durch ihre Eigenschaft, hohe Affinität zu Glykosaminoglykanen zu besitzen, an Zellen und anderen Matrices gebunden werden, wodurch sie physiologisch und pathophysiologisch möglicherweise an der Zellmigration und zellulär-proteolytischen Prozessen beteiligt ist.

Antikörper gegen FSAP können somit alle durch die FSAP vermittelten Aktivitäten beeinflussen. Für den Fall, dass Autoantikörper gegen die FSAP auftreten, kann neben einer Beeinträchtigung der physiologischen Funktionen noch hinzukommen, dass Immunkomplexe (FSAP + Antikörper) zu Nebenwirkungen bekannter Autoimmunerkrankungen betragen. Das kann beispielsweise lokalisiert am Endothel zu Vaskulitiden führen. Die Neutralisierung der FSAP-Aktivität als profibrinolytisches Agens könnte außerdem zu einem Thrombose-fördernden Zustand beitragen.

Ein weiterer Gegenstand der Erfindung ist ein diagnostisches Verfahren zum immunhistochemischen Nachweis der den Blutgerinnungsfaktor VII aktivierenden Protease, ihres Proenzyms oder ihrer Mutanten oder Spaltstücke, bei dem man auf eine Gewebeprobe einen gegen die Protease gerichteten markierten, monoklonalen oder polyklonalen Antikörper oder eines seiner Fragmente einwirken lässt, den nicht gebundenen Antikörper oder seine Fragmente auswäscht und das von dem gebundenen Antikörper oder einem seiner Fragmente ausgesendete Signal bestimmt.

Das Verfahren kann auch so durchgeführt werden, dass man auf die Gewebeprobe einen gegen die Protease, ihr Proenzym oder seine Mutanten oder Spaltstücke gerichteten unmarkierten monoklonalen oder polyklonalen Antikörper oder eines seiner Fragmente einwirken lässt, den nicht gebundenen Antikörper oder seine Fragmente auswäscht, dann einen markierten anti-Antikörper auf das Gewebe einwirken lässt und nach Auswaschen des nicht gebundenen, markierten anti-Antikörpers das von dem gebundenen anti-Antikörper oder seinen Fragmenten ausgesendete Signal bestimmt.

Es wurde außerdem gefunden, dass gegen FSAP gerichtete monoklonale oder polyklonale Antikörper, wenn sie mit chromophoren oder lumineszierenden Gruppen markiert sind, hervorragend zum Nachweis von FSAP in Gewebeschnitten humanen Ursprungs geeignet sind. Für diesen Nachweis eignen sich polyklonale Antikörper gegen FSAP, die durch Immunisierung von Kaninchen, Schafen, Ziegen oder anderen Säugetieren gewonnen werden, ebenso wie monoklonale Antikörper. Besonders gut geeignet zum histologischen, spezifischen Nachweis von FSAP, die sowohl in der aktivierten Form, als auch in der Proenzymform oder als Spaltstück vorliegen kann, sind die monoklonalen Antikörper der Hybridomazelllinien DSM ACC 2453 und DSM ACC 2454. Auch Komplexe von aktivierter FSAP mit Inhibitoren, wie Antiplasmin, können so detektiert werden. Hierfür geeignet sind alle gängigen histologischen Nachweisverfahren wie die Licht-, Fluoreszenz- und Elektronenmikroskopie.

Zum Nachweis von FSAP in den vorstehend genannten Verfahren eignen sich sowohl die vollständigen polyklonalen und monoklonalen Antikörper als auch deren Fragmente wie F(ab')₂ oder Fab, sofern sie mit einer detektierbaren Gruppe markiert sind. Dabei können die vorstehend genannten Antikörper oder ihre Fragmente allein oder als Mischung angewendet werden. Das ist besonders dann empfehlenswert, falls eines der erkannten Epitope verdeckt ist. Beispielsweise kann eine Proteindomäne durch Zellassoziation für einen Antikörper nicht zugänglich sein, wird jedoch durch einen anderen Antikörper mit der Spezifität für eine andere FSAP-Region gebunden. Auch Antikörper, die gegen die humane FSAP, deren Wildtyp und/oder gegen deren Mutanten gerichtet sind und die in der deutschen Patentanmeldung 100 52 319.6 näher beschrieben sind, können für den Nachweis von FSAP in Gewebeschnitten humanen Ursprungs eingesetzt werden.

Die bisher gewonnenen Erkenntnisse über den immunhistochemischen Nachweis von FSAP lassen sich wie folgt zusammenfassen:
- FSAP wird in fast allen der bisher untersuchten humanen Gewebe nachgewiesen;
- Endokrin-aktive Zellen wie die Leydig'schen Zwischenzellen oder die endokrinaktiven Zellen der Langerhans'schen Inseln der Pankreas lassen sich mit Antikörpern, die chromophore Gruppen tragen, sehr stark intrazytoplasmatisch anfärben;
- Epithelien und Endothelien zeigen entsprechend ihrer Lokalisation eine mehr oder weniger starke intra-zytoplasmatische lmmunreaktion mit Antikörpern gegen FSAP;
- Ganglienzellen und Dendriten der Großhirnrinde zeigen eine hohe Konzentration von FSAP, was durch eine starke immunhistologische Farbreaktion mit chromophoren Antikörpern nachgewiesen wird;
- Plasmazellen zeigen eine intensive intra-zytoplasmatische Verfärbung mit chromophoren Antikörpern;
- mesenchymale Stromazellen zeigen in komplexen Geweben keine oder nur eine schwache Farbreaktion für FSAP.

FSAP ist somit ein Protein, das als ein normaler Zellbestandteil anzusehen ist. Bislang wurde FSAP sowohl intra- als auch extrazellulär lokalisiert gefunden, wobei das erstere Kompartiment deutlich stärker anfärbbar ist. Durch die erfindungsgemäße Detektion von FSAP mit den genannten Antikörpern oder ihren Fragmenten können folgende pathologische Vorgänge erkannt werden:
- Endokrin-aktive Tumore und neuro-endokrine Tumore;
- angiogene Endothelien und Endothelien des Kapillarendothels; sowie
- angiogenaktive Tumore wie Gliome und Glioblastome, aber auch beispielsweise Gefäßtumore wie das Hämangioendothelium oder -perizyotom und Angiosarkom;
- Wundheilungsreaktionen, Granulationsgewebe und Kollagenosen;
- arteriosklerotische, (mikro)thrombosierte und nekrotisierende Areale;
- neurodegenerative Erkrankungen, wie Alzheimer'sche Krankheit, Morbus Parkinson aber auch spongiforme Enzephalitiden, beispielsweise ausgelöst durch Prionenproteine;
- Gammopathien und Myelome.

Für den Nachweis der FSAP verwendet man vorzugsweise monoklonale Antikörper der Hybridomazelllinien DSM ACC 2453 .

Das erfindungsgemäße diagnostische Verfahren wird durch das nachfolgende Beispiel näher erläutert:

Zur Untersuchung der immunhistochemischen Reaktivität der für FSAP spezifischen monoklonalen Antikörper der Hybridomazelllinie DSM ACC 2453 wurden aus adultem humanem Gewebe und malignen urologischen Tumoren 10 µm dicke Paraffinschnitte mit anschließender Entparaffinierung hergestellt, die im Zitratpuffer für 3 mal 5 Minuten in der Mikrowelle behandelt wurden. Auf diese Schnitte ließ man zunächst für 30 Minuten einen unmarkierten Antikörper der oben genannten Hybridomazelllinien einwirken. Nach Auswaschen des Gewebeschnittes ließ man auf das Gewebe einen markierten Anti-Maus-Detektionsantikörper für ebenfalls 30 Minuten einwirken und machte dann den gebundenen FSAP-Antikörper durch Bildung des APAAP-Komplexes (Alkalische Phosphatase-Anti-Alkalische-Phosphatase-Komplex) und durch Färbung mit Chromogen und Gegenfärbung mit Hämalaun sichtbar.

Als negative Kontrolle wurde in jedem Gewebe separat eine Inkubation mit dem Detektionsantikörper - ohne vorherige Inkubation mit dem FSAP-Antikörper - durchgeführt, um potentielle unspezifische Reaktionen des Detektors sichtbar zu machen. Außerdem wurde als Positivkontrolle ein Antikörper gegen α-Keratin mitgeführt.

Die Ergebnisse der immunhistochemischen Untersuchung normalen Humangewebes sind in Tabelle 1 zusammengefasst:

**Antikörper gegen FSAP und DSMZ ACC 2453 Humanes Normalgewebe**

| | **DSMZ ACC2453** | | **2453** |
|---|---|---|---|
| **Oesophagus** | | **Appendix** | |
| Plattenepithel | 2+ | Epithelien | 3+ |
| Drüsenendstücke | 0 | Muskulatur | 1+ |
| Schaltstücke | 2+ | Lymphfollikel | 2+ |
| Muskulatur | 1+ | Plasmazellen | 3+ |
| Stroma | 1+-2+ | Gefäßendothel | 1+ |
| Gefäßendothel | 2+ | **Pankreas** | |
| **Kardia (Magen)** | | Epithelien | 2+ |
| Foveolarepithel | 0 | Langerhans-Inseln | 1+ |
| Glandulae cardiacae | 2+ | Gangepithel | 2+ |
| Mucinöse Drüsenendstücke | 0 | Gefäßendothel | 1+ |
| Belegzellhaltige Drüsen | 3+ | **Speicheldrüse** | |
| Muskulatur | 1+ | Muköse Endstücke | 0 |
| Gefäßendothel | 1+ | Seröse Endstücke | 1+-2+ |
| **Korpus (Magen)** | | Schaltstücke | 1+ |
| Foveolarepithel | 0-1+ | Streifenstücke | 1 + |
| Korpus- | | Gefäßendothel | 0-1+ |
| Drüsenkörper | | | |
| Muskulatur | 1+ | **Leber** | |
| Gefäßendothel | 1+ | Hepatozyten | 2+ |
| **Duodenum** | | Gallengänge | 0 |
| Epitehlien | 1+ | Gefäßendothel | (1+) |
| Brunnersche Drüsen | 0 | **Gallenblase** | |
| Muskulatur | O-1+ | Epithel | 1+ |
| Lymphfollikel | 2+ | Muskulatur | 1+ |
| Ganglienzellen | 3+ | Gefäßendothel | 1+ |
| Gefäßendothel | 1+ | **Ductus cysticus** | |
| **Dünndarm** | | Epithel | 3+ |
| Epithelien | 3+ | Muskulatur | 1+ |
| Muskulatur | 1+ | Ganglienzellen | 3+ |
| Stroma | 2+ | Gefäßendothel | 2+ |
| Ganglienzellen | 3+ | **Hoden** | |
| Gefäßendothel | 1+ | Leydigsche Zwischenzellen | 1+ |
| **Kolon/Rektum** | | Sertoll-Zellen | 1+ |
| Epithelien | 1+ | Keimzellen | 1+ |
| Lymphfollikel | 1+ | Gefäßendothel | 1+ |
| Plasmazellen | 1+ | **Rete testis** | |
| Gefäßendothel | 0 | Epithel | 2+ |
| | | | |
| | **2453** | | **2453** |
| **Nebenhoden** | | **Plazenta** | |
| Ductus epididymidis | 2+ | Chorionepithel | 2+ |
| Ductulus efferens | 2+ | Amnionepithel | 2+ |
| Stroma | 1+ | Deziduazellen | 2+ |
| Gefäßendothel | 1+ | Stromazellen | +/- |
| **Samenblase** | | Gefäßendothel | 1+ |
| Epithel | 3+ | **Eihäute** | |
| Muskulatur | 1+ | Amnionepithel | 2+ |
| Gefäßendothel | 2+ | Deziduazellen | 1+ |
| **Ductus deferens** | | Fibroblasten | 1+-2+ |
| Epithel | 3+ | **Cervix Uteri** | |
| Längsmuskelschic ht | +/- | Drüsenepithel | 0 |
| Ringmuskelschicht | 3+ | Gefäßendothel | 0-1 |
| Gefäßendothel | 3+ | Stroma | 0-1 |
| **Prostata** | | **Tube uterina** | |
| Drüsenepithel | 2+ | Epithel | 3+ |
| Muskulatur | 1+ | Muskulatur | 1+ |
| Gefäßendothel | 1+-2+ | Gefäßendothel | 2+ |
| **Niere** | | **Mamma** | |
| Tubuli | 1+ | Epithelien Brustdrüsenlobuli | 2+ |
| Glomerula | 0 | Gangepithel Aus-führungsgänge | 2+ |
| Markepithel | 1+ | Fibroblasten | 1+ |
| Gefäßendothel | 0-1+ | Plasmazellen | 2+ |
| **Harnblase** | | Gefäßendothel | 0 |
| Urothel | 1+-2+ | **Schilddrüse** | |
| Muskulatur | 1+ | Follikelepithel | 1+-2+ |
| Plasmazellen | 2+ | Stroma | 1+ |
| Fibroblasten | 1+-2+ | Gefäßendothel | 0 |
| Peripherer Nerv | 0 | **Thymus** | |
| **Nebenniere** | | Hassal-Körperchen | 2+ |
| Zona glomerulosa | 1+ | Follikel | 2+ |
| Zona fasciculata | (1+) | Mantelzone | (1+) |
| Zona reticularis | (1+) | Stemhimmelmakrophage n | 1+ |
| Mark | 0 | **Milz** | + |
| Gefäßendothel | (1+) | **Tonsille** | +/- |
| **Endometrium** | | **Lymphknoten** | +/- |
| Drüsenepithel | 2+ | **Kieferhöhle** | |
| Stromazellen | 1+ | Respiratorisches Epithel | 2+ |
| Myometrium | 1+ | Plasmazellen | 3+ |
| Gefäßendothel | 2+ | Gefäßendothel | 1+ |
| | | | |
| | **2453** | | **2453** |
| **Lunge** | | **Fettgewebe** | 2+ |
| Bronchialepithel | 1+ | Gefäßendothel | 2+ |
| Alveolarepithel | 1+ | **Haut** | |
| Bronchialdrüsen | 1+ | Epidermis | 1+-2+ |
| Knorpel | | Dermis | 0 |
| Muskulatur | 1+ | Subkutis | 0 |
| Alveolarmakrophagen | 2+ | Schweißdrüsen | 0 |
| Elastische Fasern | 2+-3+ | Gefäßendothel | 0 |
| Gefäßendothel | 1+ | **Endokard** | 0 |
| **Skelettmuskulatur** | 1+ | Fibroblasten | 2+-3+ |

| | | | |
|---|---|---|---|
| 0 = negativ 1+ = schwach positiv 2+ = mäßig stark positiv 3+ = stark positiv | | | |

Bei endokrinen Zellen wie den Langerhans'schen Inseln des Pankreas, den Leidig'schen Zwischenzellen des Hodeninterstitiums, bei den Deziduazellen der Plazenta und dem beleghaltigen Drüsenkörper der Magenkardia sowie dem hochzylindrischen Epithel des Ductus cysticus zeigt sich eine starke, teils feingranuläre Reaktion. Stark positive Reaktionen wurden in Gewebeverbänden gelegenen Plasmazellen und gangligen Zellen und den Nervenzellen der Großhirnrinde beobachtet. Auch die dezidualen Zellen, das Amnioepithel, und die Fibroplasten der Eihäute zeigten eine sehr starke immunhistologische Anfärbbarkeit, wie auch das auskleidende Epithel der Samenbläschen und die Enterozyten des Dünndarms.

Untersuchungen Formalin-fixierten, in Paraffin eingebetten Tumormaterials urologischer Tumoren zeigten eine schwache bis mäßig starke intra-zytoplasmatische Reaktion unterschiedlich differenzierter Adenokarzinome der Prostata. Tumorzellen seminomatöser Hodentumore zeigten nur eine schwache intra-zytoplasmatische Reaktion, während nicht-seminomatöse Tumore (Embryokarzinome und Chorionkarzinome) eine deutlich verstärkte Anfärbbarkeit der Tumorzellen aufwiesen, die auf erhöhte Konzentrationen von FSAP hinwiesen.

Das erfindungsgemäße diagnostische Verfahren erlaubt somit einen immunhistochemischen Nachweis von pathologischen Vorgängen in den verschiedensten Organen.

## Patentansprüche

1. Hybridomazelllinie DSM ACC2453.

2. Monoklonaler Antikörper gegen die den Blutgerinnungsfaktor VII aktivierende Protease (FSAP) **dadurch gekennzeichnet, dass** er von der Hybridomazelliinie gemäß Anspruch 1 gebildet wird.

3. In vitro Verfahren zum Nachweis der FSAP oder ihres Proenzyms mit einem Immunoassay **dadurch gekennzeichnet, dass** man einen monoklonalen Antikörper gemäß Anspruch 2 oder antigen-bindende Fragmente des Antikörpers, bevorzugt Fab- oder F(ab')₂-Fragmente verwendet.

4. In vitro Verfahren zum Nachweis der FSAP oder ihres Proenzyms gemäß Anspruch 3, bei dem man eine Probe, die die Protease oder ihr Proenzym enthalten soll, mit einem auf einem festen Träger fixierten ersten Antikörper inkubiert und auswäscht, dann einen zweiten markierten Antikörper zugibt und abermals auswäscht und das vom zweiten Antikörper hervorgerufene Signal misst.

5. In vitro Verfahren zum Nachweis der FSAP oder ihres Proenzyms gemäß Anspruch 3, bei dem man die in der zu untersuchenden Probe enthaltene FSAP oder ihr Proenzym auf einem Träger fixiert und sie mit einem markierten Antikörper gemäß Anspruch 2 und anschließendem Nachweis des monoklonalen Antikörpers detektiert.

6. In vitro Verfahren zum Nachweis der FSAP oder ihres Proenzyms gemäß Anspruch 3, bei dem man einen auf einen Träger fixierten Antikörper mit einer auf die FSAP oder ihr Proenzym zu untersuchenden Probe in Gegenwart einer markierten Protease oder ihres Proenzyms versetzt und das durch die Markierung hervorgerufene Signal misst.

7. In vitro Verfahren zum Nachweis der FSAP oder ihres Proenzyms in einem Western Blot-Verfahren **dadurch gekennzeichnet, dass** man einen monoklonalen Antikörper gemäß Anspruch 2 oder antigen-bindende Fragmente des Antikörpers, bevorzugt Fab- oder F(ab')₂-Fragmente verwendet.

8. In vitro Verfahren zum immunhistochemischen Nachweis der FSAP, ihres Proenzyms, oder ihrer Spaltstücke **dadurch gekennzeichnet, dass** man einen monoklonalen Antikörper gemäß Anspruch 2 oder antigen-bindende Fragmente des Antikörpers, bevorzugt Fab- oder F(ab')₂-Fragmente verwendet.

9. In vitro Verfahren gemäß Anspruch 8 zum immunhistochemischen Nachweis der FSAP, ihres Proenzyms, ihrer Mutanten oder ihrer Spaltstücke in Gewebeproben, insbesondere in Gewebeschnitten humanen Ursprungs.

10. In vitro Verfahren zum Nachweis der FSAP, ihres Proenzyms oder ihrer Spaltstücke gemäß den Ansprüchen 3 bis 9 **dadurch gekennzeichnet, dass** man einen monoklonalen Antikörper gemäß Anspruch 2 oder antigen-bindende Fragmente des Antikörpers verwendet, wobei die Antikörper oder ihre antigenbindenden Fragmente mit einer radioaktiven oder fluoreszierenden oder einer enzymatisch aktiven Substanz markiert sind.

11. In vitro Verfahren zur Bestimmung der Aktivität der FSAP oder ihres Proenzyms **dadurch gekennzeichnet, dass** man einen monoklonalen Antikörper gemäß Anspruch 2 oder antigen-bindende Fragmente des Antikörpers, bevorzugt Fab- oder F(ab')₂-Fragmente verwendet.

12. In vitro Verfahren zur Bestimmung der Aktivität der FSAP oder ihres Proenzyms gemäß Anspruch 11, bei dem man die die FSAP enthaltende Probe an einem festen Träger inkubiert, an den zuvor ein Antikörper gemäß Anspruch 2 oder antigen-bindende Fragmente des Antikörpers gekoppelt wurde, und nach Auswaschen des festen Trägers die daran fixierte FSAP mit Reagenzien inkubiert, die deren Aktivitätsbestimmung erlauben.

13. Verwendung eines Antikörpers gemäß Anspruch 2 zum Nachweis und zur Bestimmung der Aktivität von FSAP und ihren Mutanten in vitro.

14. In vitro Verfahren zum Nachweis von Antikörpern gegen die FSAP oder ihr Proenzym oder eine durch den Austausch einer oder mehrerer Aminosäuren gebildeten Mutante der FSAP, wobei man eine Probe, die die Antikörper enthalten könnte, auf die an einen festen Träger fixierte FSAP und /oder FSAP-Mutante einwirken lässt, danach wäscht und die an die Protease gebundenen Antikörper nachweist, **dadurch gekennzeichnet dass** der feste Träger mit einem Antikörper gemäß Anspruch 2 beschichtet ist, der mit der FSAP und/oder ihrer Mutante beladen ist.

15. Verfahren zur Reindarstellung der FSAP oder ihres Proenzyms **dadurch gekennzeichnet, dass** man einen monoklonalen Antikörper gemäß Anspruch 2 an einen Träger koppelt, diesen mit einer die Protease oder ihr Proenzym enthaltenden Flüssigkeit äquilibriert, anschließend auswäscht und dann die Protease oder ihr Proenzym gewinnt.

## Claims

1. Hybridoma cell line DSM ACC2453.

2. Monoclonal antibody to coagulation factor VII-activating protease (FSAP), **characterized in that** it is produced by the hybridoma cell line according to Claim 1.

3. In-vitro method of detecting FSAP or its pro-enzyme using an immunoassay, **characterized in that** a monoclonal antibody according to Claim 2 or antigen-binding fragments of the antibody, preferably Fab or F(ab')₂ fragments, are employed.

4. In-vitro method of detecting FSAP or its pro-enzyme according to Claim 3, which method comprises incubating a sample supposed to contain the protease or its pro-enzyme with a first antibody fixed to a solid support which is then washed out, and thereafter adding a second, labelled antibody and washing out again and measuring the signal produced by the second antibody.

5. In-vitro method of detecting FSAP or its pro-enzyme according to Claim 3, which method comprises fixing the FSAP present in the sample to be tested or its pro-enzyme to a support and detecting it using a labelled antibody according to Claim 2 and subsequently detecting the monoclonal antibody.

6. In-vitro method of detecting FSAP or its pro-enzyme according to Claim 3, which method comprises exposing an antibody fixed to a support to a sample to be tested for FSAP or its pro-enzyme in the presence of a labelled protease or its pro-enzyme and measuring the signal produced by the label.

7. In-vitro method of detecting FSAP or its pro-enzyme by Western blotting, **characterized in that** a monoclonal antibody according to Claim 2 or antigen-binding fragments of the antibody, preferably Fab or F(ab')₂ fragments, are employed.

8. In-vitro method of immunohistochemical detection of FSAP, its pro-enzyme or its cleavage products, **characterized in that** a monoclonal antibody according to Claim 2 or antigen-binding fragments of the antibody, preferably Fab or F(ab')₂ fragments, are employed.

9. In-vitro method according to Claim 8 of immunohistochemical detection of FSAP, its pro-enzyme, its mutants or its cleavage products in tissue samples, in particular in tissue samples of human origin.

10. In-vitro method of detecting FSAP, its pro-enzyme or its cleavage products according to Claims 3 to 9, **characterized in that** a monoclonal antibody according to Claim 2 or antigen-binding fragments of the antibody are employed, wherein the antibodies or their antigen-binding fragments are labelled with a radioactive or fluorescent or enzymatically active substance.

11. In-vitro method of determining the activity of FSAP or of its pro-enzyme, **characterized in that** a monoclonal antibody according to Claim 2 or antigen-binding fragments of the antibody, preferably Fab or F(ab')₂ fragments, are employed.

12. In-vitro method of determining the activity of FSAP or of its pro-enzyme according to Claim 11, which method comprises incubating the FSAP-containing sample on a solid support to which an antibody according to Claim 2 or antigen-binding fragments of the antibody have been coupled earlier and, after washing out the solid support, incubating the FSAP fixed thereto with reagents which allow FSAP activity to be determined.

13. Use of an antibody according to Claim 2 for detecting FSAP and its mutants and determining the activity thereof in vitro.

14. In-vitro method of detecting antibodies to FSAP or to its pro-enzyme or to an FSAP mutant produced by substitution of one or more amino acids, wherein a sample possibly containing the antibodies is allowed to act on FSAP and/or the FSAP mutant, both of which are fixed to a solid support, and then, after washing, the antibodies bound to the protease are detected, **characterized in that** the solid support is coated with an antibody according to Claim 2, which is loaded with FSAP and/or its mutant.

15. Method of purifying FSAP or its pro-enzyme, **characterized in that** a monoclonal antibody according to Claim 2 is coupled to a support which is equilibrated with a liquid containing the protease or its pro-enzyme and subsequently washed out, and the protease or its pro-enzyme is then recovered.

## Revendications

1. Lignée d'hybridomes DSM ACC2453.

2. Anticorps monoclonal dirigé contre la protéase activant le facteur de coagulation VII (FSAP), **caractérisé en ce qu'**il est formé par la lignée d'hybridomes selon la revendication 1.

3. Procédé in vitro pour la détection de la FSAP ou de sa pro-enzyme à l'aide d'un dosage immunologique, **caractérisé en ce qu'**on utilise un anticorps monoclonal selon la revendication 2 ou des fragments de l'anticorps qui se lient à l'antigène, de préférence des fragments Fab ou F(ab')₂.

4. Procédé in vitro pour la détection de la FSAP ou de sa pro-enzyme selon la revendication 3, dans lequel on met un échantillon qui doit contenir la protéase ou sa pro-enzyme à incuber avec un premier anticorps fixé sur un support solide et on lave, puis on ajoute un deuxième anticorps marqué et on lave de nouveau et on mesure le signal provoqué par le deuxième anticorps.

5. Procédé in vitro pour la détection de la FSAP ou de sa pro-enzyme selon la revendication 3, dans lequel on fixe sur un support la FSAP ou sa pro-enzyme, contenue dans l'échantillon à analyser, et on la détecte à l'aide d'un anticorps marqué selon la revendication 2 et par détection subséquente de l'anticorps monoclonal.

6. Procédé in vitro pour la détection de la FSAP ou de sa pro-enzyme selon la revendication 3, dans lequel on ajoute à un anticorps fixé sur un support un échantillon à analyser quant à la présence de la FSAP ou de sa pro-enzyme, en présence d'une protéase marquée ou de sa pro-enzyme et on mesure le signal provoqué par le marquage.

7. Procédé in vitro pour la détection de la FSAP ou de sa pro-enzyme dans un procédé western-blot, **caractérisé en ce qu'**on utilise un anticorps monoclonal selon la revendication 2 ou des fragments de l'anticorps qui se lient à l'antigène, de préférence des fragments Fab ou F(ab')₂.

8. Procédé in vitro pour la détection immunohistochimique de la FSAP, de sa pro-enzyme ou de ses fragments, **caractérisé en ce qu'**on utilise un anticorps monoclonal selon la revendication 2 ou des fragments de l'anticorps qui se lient à l'antigène, de préférence des fragments Fab ou F(ab')₂.

9. Procédé in vitro selon la revendication 8 pour la détection immunohistochimique de la FSAP, de sa pro-enzyme, de ses mutants ou de ses fragments dans des échantillons de tissu, en particulier dans des coupes de tissu d'origine humaine.

10. Procédé in vitro pour la détection de la FSAP, de sa pro-enzyme ou de ses fragments selon les revendications 3 à 9, **caractérisé en ce qu'**on utilise un anticorps monoclonal selon la revendication 2 ou des fragments de l'anticorps qui se lient à l'antigène, les anticorps ou leurs fragments se liant à l'antigène étant marqués par une substance radioactive ou fluorescente ou une substance à activité enzymatique.

11. Procédé in vitro pour la détermination de l'activité de la FSAP ou de sa pro-enzyme, **caractérisé en ce qu'**on utilise un anticorps monoclonal selon la revendication 2 ou des fragments de l'anticorps qui se lient à l'antigène, de préférence des fragments Fab ou F(ab')₂.

12. Procédé in vitro pour la détermination de l'activité de la FSAP ou de sa pro-enzyme selon la revendication 11, dans lequel on met l'échantillon contenant la FSAP à incuber sur un support solide auquel a été préalablement couplé un anticorps selon la revendication 2 ou des fragments de l'anticorps qui se lient à l'antigène, et après avoir lavé le support solide, on met la FSAP fixée sur celui-ci à incuber avec des réactifs qui permettent la détermination de son activité.

13. Utilisation d'un anticorps selon la revendication 2, pour la détection et pour la détermination de l'activité de la FSAP et de ses mutants in vitro.

14. Procédé in vitro pour la détection d'anticorps dirigés contre la FSAP ou sa pro-enzyme ou un mutant de la FSAP formé par le remplacement d'un ou plusieurs aminoacides, dans lequel on laisse agir un échantillon, susceptible de contenir les anticorps, sur la FSAP et/ou des mutants de FSAP, fixés sur un support solide, puis on lave et on détecte les anticorps liés à la protéase,
**caractérisé en ce que** le support solide est tapissé avec un anticorps selon la revendication 2, qui est chargé avec la FSAP et/ou ses mutants.

15. Procédé pour la préparation à l'état pur de la FSAP ou de sa pro-enzyme, **caractérisée en ce qu'**on couple un anticorps monoclonal selon la revendication 2 à un support, on le met en contact avec un liquide contenant la protéase ou sa pro-enzyme, puis on lave et on recueille ensuite la protéase ou sa pro-enzyme.
